(19) Europäisches Patentamt
European Patent Office
Office européen des brevets

(11) Numéro de publication: **0 674 185 A1**

(12) **DEMANDE DE BREVET EUROPEEN**

(21) Numéro de dépôt: **95200679.9**

(51) Int. Cl.[6]: **G01S 15/89**, A61B 8/06

(22) Date de dépôt: **21.03.95**

(30) Priorité: **25.03.94 FR 9403557**

(43) Date de publication de la demande: **27.09.95 Bulletin 95/39**

(84) Etats contractants désignés: **AT CH DE DK FR GB LI**

(71) Demandeur: **LABORATOIRES D'ELECTRONIOUE PHILIPS**
**22, Avenue Descartes**
**F-94450 Limeil-Brévannes (FR)**
(84) **FR**

(71) Demandeur: **PHILIPS ELECTRONICS N.V.**
**Groenewoudseweg 1**
**NL-5621 BA Eindhoven (NL)**
(84) **CH DE DK GB LI AT**

(72) Inventeur: **Bonnefous, Odile**
**Société Civile S.P.I.D.,**
**156, Boulevard Haussmann**
**F-75008 Paris (FR)**

(74) Mandataire: **Pyronnet, Jacques**
**Société Civile S.P.I.D.**
**156, Boulevard Haussmann**
**F-75008 Paris (FR)**

(54) **Procédé et appareil de détection et de caractérisation d'un segment d'artère par échographie ultrasonore.**

(57) L'échographe utilisé, du type à traitement de signal dans le domaine temporel avec intercorrélation-interpolation des signaux échographiques, est adapté pour la mesure de vitesses de déplacement radiales des parois d'une artère. Selon l'invention, une trame est balayée selon une péiode T de quelques ms, à raison d'un tir par ligne, et les intercorrélations-interpolations sont effectuées entre lignes de tir colinéaires décalées de la période T (52), ce qui permet d'obtenir des valeurs de vitesses v(x,z,t) dans une pemière mémoire (55), et d'amplitudes A(x,z,t) dans une deuxième mémoire (57). Ces valeurs sont ensuite traitées (60) pour en déduire des courbes de dilatation et de compression.

Application à la caractérisation des sténoses.

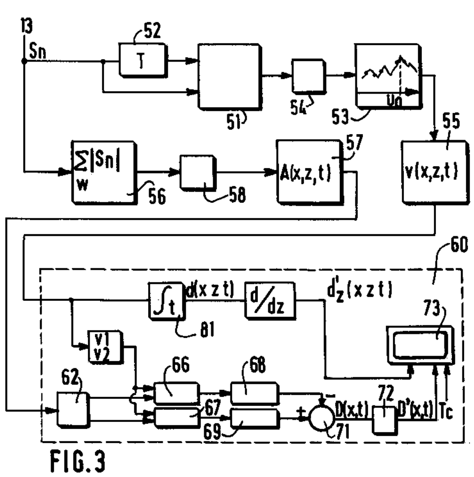


FIG.3

EP 0 674 185 A1

Rank Xerox (UK) Business Services
(3.10/3.09/3.3.4)

La présente invention concerne un procédé de détection et de caractérisation d'un segment d'artère par échographie ultrasonore à partir d'une barrette de transducteurs ultrasonores, engendrant un plan de coupe ou trame constitué par L lignes de tir parallèles, successives dans une direction $\vec{z}$ perpendiculaire à l'axe $\vec{x}$ de l'artère, associée à un circuit d'émission et à un circuit de réception, ladite échographie étant du type à traitement de signal dans le domaine temporel avec intercorrélation des signaux échographiques relatifs auxdites lignes et interpolation des signaux intercorrélés pour la détermination des vitesses radiales v des points dudit plan de coupe limités à la traversée des parois de l'artère et à ses environs immédiats.

L'invention concerne aussi, pour la mise en oeuvre de ce procédé, un appareil pour la détection et la caractérisation d'un segment d'artère comportant un échographe ultrasonore et une station de travail, l'échographe consistant en une sonde munie d'une barrette de transducteurs ultrasonores, conçue pour émettre une trame de L lignes successives selon une période T dans une direction $\vec{z}$ perpendiculaire à l'axe $\vec{x}$ de l'artère, un circuit d'émission et un circuit de réception et étant du type à traitement de signal dans le domaine temporel et comportant à cet effet des moyens d'intercorrélation et des moyens d'interpolation des signaux échographiques.

De tels procédé et appareil, utilisables dans le domaine médical, sont d'un intérêt tout particulier pour la caractérisation d'une sténose dont la détection a pu être faite préalablement soit par angiographie, soit au moyen de la sonde ultrasonore elle-même.

Un échographe est un appareil d'examen de milieux, utilisant le rayonnement ultrasonore comme source d'information. Un tel appareil met en oeuvre, pour son fonctionnement, une étape d'émission, par tirs périodiques, de signaux ultrasonores vers le milieu exploré ainsi qu'une étape de réception et traitement des échos renvoyés par les obstacles rencontrés dans le milieu exploré. Les deux étapes sont réalisées avec la même sonde ultrasonore en contact avec le milieu. Cette sonde est une structure composée en général de tout un réseau de transducteurs ultrasonores assemblés sous forme d'une barrette linéaire. Elle émet à une fréquence centrale fo de l'ordre de quelques MHz.

Au cours de l'étape d'émission, on explore sélectivement le milieu selon une ligne. En réception, l'image de cette ligne explorée est formée en tenant compte du temps de vol dans le milieu et de l'amplitude des échos issus des différents obstacles rencontrés sur la ligne. L'image d'un plan de coupe est réalisée par un balayage de cette ligne. Pour obtenir une bonne résolution d'image, on cherche à explorer le milieu de façon très sélective par un tir ultrasonore focalisé, et, en réception à sélectionner les échos issus de la même ligne en utilisant une ouverture focalisante.

Une technique de focalisation couramment employée consiste à utiliser un réseau linéaire de transducteurs et à définir à l'émission un faisceau incident focalisé à l'aide d'une loi de retards imposée aux impulsions d'excitation des transducteurs. En réception, la focalisation est alors réalisée de façon similaire, en retardant de façon appropriée les signaux reçus par chacun des transducteurs du réseau, avant leur sommation et les traitement ultérieurs. Ce traitement des signaux en réception, qui conduit à disposer d' un signal de grande amplitude pour les échos issus du point de focalisation (ce point est situé sur la ligne explorée) et de signaux faibles pour tous les autres échos, est, habituellement appelé formation de voie.

Pour obtenir une bonne focalisation sur toute une ligne, les échographes actuels utilisent une loi de focalisation en réception qui n'est en général pas continue mais par zones de l'ordre de grandeur du centimètre (cm) dans la direction $\vec{z}$ du tir. A l'émission, on émet dans la même direction plusieurs faisceaux focalisés à différentes profondeurs successivement. On notera en conséquence, pour ce qui suit, que lorsque la partie à analyser, à l'intérieur d'un organisme est petite, de l'ordre de 2 cm sur 3 cm par exemple, une seule zone de focalisation peut s'avérer suffisante et on peut donc n'émettre qu'une fois, ce qui permet d'accroître la fréquence trame d'analyse.

L'utilisation de réseaux linéaires d'éléments transducteurs apporte aussi la possibilité non seulement de focaliser mais aussi d'effectuer le balayage nécessaire à la formation de l'image bidimensionnelle qui apparaît sur l'écran d'un moniteur échographique classique (imagerie 2D). Pour l'examen d'un segment d'artère de largeur de l'ordre de 3 cm, par exemple, on peut prévoir une trame de 128 lignes espacées de 0,25 mm.

Pour en revenir à l'examen non invasif d'une artère en fonctionnement, on observe que la détection et l'évaluation des sténoses, quant à leur origine et à leur gravité, sont un but important recherché pour les applications médicales des ultrasons. Des mesures utilisant la technique Doppler pour les échos ultrasonores constituent le premier moyen d'investigation utilisé par les médecins à cet effet. Cependant, il apparaît que les diagnostics médicaux résultent de critères peu objectifs, tels que le pourcentage de réduction de la section utile de l'artère, ou du gradient de pression estimé à partir de la mesure Doppler de la vitesse du sang dans la sténose. Bien que ces méthodes soient cliniquement reconnues, il semble qu'elles soient seulement des moyens de fortune, peu précis.

Après détection d'une sténose, le médecin doit décider si une intervention chirurgicale est nécessaire ou bien si une simple angioplastie (dilatation locale) conviendrait. Cette dernière est pratiquée si la sténose réduit la section utile de l'artère d'un pourcentage qui ne dépasse pas 70 %, alors que la première solution est décidée lorsque le vaisseau est presque bouché. Parallèlement aux détections morphologiques par ultrasons ou angiographie, la vitesse du sang est le paramètre préféré, qu'elle soit mesurée par des sytèmes Doppler, ou des systèmes CVI (Colour Velocity Imaging en anglais), comme décrit notamment dans la revue annuelle L.E.P. (Laboratoires d'Electronique Philips) 1990, dans l'article : "De l'algorithme au produit", pages 43, 44, par 0. Bonnefous.

L'équation de Bernoulli simplifiée : $V^2 = \Delta P$ (V et P étant respectivement la vitesse et la pression sanguine) est supposée donner une indication du gradient de pression à travers la sténose. Cependant, cette caractérisation n'est pas assez spécifique, pour la raison probable que l'équation de Bernoulli utilisée ne correspond pas au phénomène de propagation qui décrit les relations existant entre 1a pression et le débit sanguin dans le réseau artériel. Les médecins sont conduits à ranger les sténoses dans deux classes, les unes étant qualifiées de : significativement hémodynamiques, et les autres de : non significativement hémodynamiques, à défaut de pouvoir quantifier l'altération du vaisseau. De plus, il y a une contradiction apparente entre le fait que la vitesse sanguine s'accroît dans la sténose à cause et à mesure de la réduction de la section utile et le fait que cette vitesse décroît ensuite brusquement lorsque cette section utile décroît au point d'engendrer une thrombose (artère presque bouchée). Une analyse mathématique plus fine est développée plus loin, qui permet de rendre compte de ces étranges comportements hémodynamiques et qui constitue une nouvelle approche pour la caractérisation des sténoses. On verra ci-après que les moyens proposés selon l'invention pour l'observation et les mesures y associées en conformité avec des acquisitions in vivo concernant des cas normaux et pathologiques, répondent bien à cette nouvelle approche de caractérisation des sténoses.

Depuis plus de 10 ans des recherches sont effectuées dans le domaine de l'échographie ultrasonore pour savoir s'il est possible de caractériser avec précision le mouvement pulsatile, en particulier d'un segment d'artère donné, notamment dans le but de savoir si l'on pourraît ainsi caractériser une partie de l' artère présentant un rétrécissement (angustie, sténose, athérosclérose). A ce sujet on peut citer la demande de brevet FR-A-2 563 991 qui se propose de rechercher, pour deux lignes de tir traversant un vaisseau, les limites de ce vaisseau et la variation de ces limites au cours du temps, ce qui résulte en deux courbes de dilatation légèrement décalées dans le temps, ce décalage étant représentatif du temps de transmission de l'onde de pression, entre les deux lignes de tir, à l'intérieur du vaisseau. Pour cette manipulation, un scanographe ultrasonique à effet Doppler est utilisé. Or un tel principe de mesure des échos manque de finesse et les dispositions adoptées pour déterminer la position des parois du vaisseau sont peu précises. D'autre part, il n'est pas possible de construire plus de deux courbes de dilatation au cours du même cycle cardiaque, ce qui est une source d'irrégularités lors du réarrangement des courbes représentatives du segment de vaisseau analysé.

Le but de la présente invention est de caractériser un segment d'artère saine ou sténosée à partir d'informations précises recueillies in vivo sur ce segment d'artère selon des lignes de tir nombreuses et rapprochées, pendant la durée d'un cycle cardiaque, en utilisant un matériel échographique basé sur le traitement du signal dans le domaine temporel avec intercorrélation-interpolation des signaux.

Un autre but est de déterminer, pour un segment d'artère sténosée, une valeur de fréquence de coupure fc en deçà de laquelle la fréquence cardiaque fondamentale ainsi que ses premiers harmoniques ne sont pas transmis à travers cette dernière, seuls les harmoniques de fréquence plus élevés, de fréquence supérieure à fc étant transmis, avec un certain amortissement.

Ces buts sont atteints et les inconvénients de l'art antérieur sont atténués grâce au fait que le procédé défini au premier paragraphe est remarquable en ce que, ladite trame étant balayée selon une période T de quelques ms, à raison d'un tir par ligne, lesdites intercorrélation et interpolation sont effectuées entre lignes de tir colinéaires décalées de la période T, afin d'obtenir au moins pour la durée d'un cycle cardiaque, dans une première mémoire une première séquence d'images en $\vec{x}$ et $\vec{z}$ de valeurs de vitesse v(x,z,t), dans une deuxième mémoire une deuxième séquence d'images en $\vec{x}$ et $\vec{z}$ de valeurs d'amplitudes A(x,z,t), et que lesdites valeurs de vitesse et d'amplitude sont ensuite exploitées sur station de travail pour en déduire, par intégration temporelle des valeurs de vitesses, la courbe de dilatation artérielle le long de chacune des L lignes de trame pendant au moins la durée Tc d'un cycle cardiaque.

Un mode de réalisation avantageux du procédé est remarquable en ce que les opérations suivantes sont effectuées par la station de travail :

a) le seuillage des amplitudes pour identifier les 2 parois artérielles diamétralement opposées,

b) le moyennage spatial selon $\vec{z}$ des vitesses dans chaque paroi,

c) l'intégration temporelle des deux vitesses ainsi moyennées avec correction des mouvements d'ensemble pour en déduire des valeurs de dilatation D(x,t) des parois, et des courbes de dilatation artérielle le long de chacune des L lignes de la trame, ces courbes étant calées sur la période Tc du cycle cardiaque,

d) le recalage en phase desdites courbes de dilatation nécessité par les décalages temporels imposés aux L lignes de chaque trame.

Pour la mise en oeuvre du procédé précité, on peut utiliser notamment l'appareil décrit dans la demande de brevet EP 0 225 667 (incorporée ici par référence) au nom de la demanderesse (PHF 85.593), qui fonctionne sur le principe de la corrélation temporelle (1 bit), et qui donne des mesures de vitesses précises car indépendantes de la fréquence ultrasonore. Dans cet appareil, l'unité de mesure de la vitesse d' écoulement sanguin notamment comporte, dans une voie de traitement numérique, un circuit d'intercorrélation qui délivre, à partir de deux lignes de tir successives dans le temps mais colinéaires selon un axe de tir donné, des valeurs de fonction de corrélation, et un circuit de multiplexage-interpolation fournissant à partir desdites valeurs des fonctions de corrélation une estimée de la vitesse V(t,z) des milieux traversés, c'est-à-dire des profils de vitesses instantanées selon l'axe de tir. En l'occurrence, il s'agit de déplacement sanguin, ce qui nécessite un dispositif de suppression d'échos fixes, c'est-à-dire les échos très forts dus aux parois artérielles, dont les vitesses de déplacement, très faibles, peuvent être considérées comme négligeables, étant de l'ordre de 100 fois plus faibles. Plus généralement et lorsque ce n'est pas le flux sanguin qui est particulièrement visé, un suppresseur d'échos fixes n'est pas nécessaire, comme c'est le cas pour la mise en oeuvre de la présente invention où l'on s'intéresse aux déplacements radiaux des parois d'une artère.

Par ailleurs une contrainte qui peut limiter l'utilisation d'un échographe est celle dite de détermination biaisée (mieux connue sous le vocable anglais d'aliasing) qui empêche l'estimation de vitesses absolues supérieures à une certaine vitesse limite :

$$Vlim = \frac{C_{us}}{4} \frac{F}{fo} \qquad (0)$$

où $C_{us}$ représente la vitesse de propagation de l'onde ultrasonore et F la fréquence de récurrence de l'excitation des transducteurs. Ce phénomène est lié à l'indétermination induite par la périodicité du signal échographique. Une description détaillée en est donnée dans l'ouvrage : "Doppler Ultrasound and Its Use in Clinical Measurement", P. Atkinson and J.P. Woodcock, Academmic Press, 1982.

A titre d'exemple, avec une période de récurrence 1/F de 100 µs, une fréquence acoustique centrale fo de 5 MHz et une vitesse de propagation $C_{us}$ 1500 m/s, on obtient une vitesse limite de 75 cm/s qui convient la plupart du temps pour la mesure de vitesses sanguines. A contrario, en remarquant que les déplacements artériels sont environ 100 fois moins rapides que ceux des globules sanguins, il devient possible de multiplier dans un rapport 100 la période de récurrence 1/F en la portant à 10 ms environ, toutes choses égales par ailleurs, c'est-à-dire sans diminuer la fréquence fo, ce qui réduirait la précision de la mesure et la résolution.

L'idée de base de l'invention est de mettre à profit la possibilité d'augmenter cette période de récurrence de tirs colinéaires, pour la rendre égale à une période de trame. Il s'agit certes d'une trame réduite, d'une centaine de lignes et de faible portée, à raison d'une seule focalisation en émission par ligne, comme on l'a vu plus haut, soit quelques centimètres carrés, mais ceci est suffisant pour l'analyse d'une sténose. Il devient ainsi possible de créer un nouveau mode d'imagerie mettant en évidence de façon très précise les mouvements des parois d'un segment d'artère, l'information instantanée ainsi obtenue étant par ailleurs mise sous forme numérique et donc d'un emploi très souple, notamment pour différents types de représentation en $\vec{x}$, $\vec{z}$, t au moyen d'une station de travail, à la portée de l'homme de métier.

Dans ces conditions, un appareil tel que décrit au deuxième paragraphe, pour la mise en oeuvre des procédés indiqués ci-dessus, est remarquable en ce que ledit circuit d'émission comporte des premiers moyens pour effectuer un seul tir par ligne de trame, que ledit circuit de réception comporte des deuxièmes moyens pour effectuer lesdites intercorrélations et interpolations entre lignes de tirs colinéaires décalées de la période T, qu'il comporte en outre une première mémoire de séquence d'images en $\vec{x}$ et $\vec{z}$ de valeurs de vitesses v(x,z,t) et une deuxième mémoire de séquences d'images en $\vec{x}$ et $\vec{z}$ de valeurs d'amplitudes, A(x,z,t), pendant la durée d'au moins un cycle cardiaque, et que ladite station de travail comporte des moyens pour exploiter le contenu desdites première et deuxième mémoires et en déduire des valeurs d'élongation ainsi que des courbes de dilatation pour le segment d'artère analysé.

Un mode de réalisation avantageux de l'appareil est remarquable en ce que ladite station de travail comporte :

a) des moyens de seuillage pour seuiller lesdites amplitudes A(x,z,t) selon $\vec{z}$ afin d'identifier les 2 parois artérielles,

b) des moyens de moyennage pour effectuer un moyennage spatial selon $\vec{z}$ de l'information de vitesse v(x,z,t) dans chaque paroi,

c) des moyens d'intégration pour effectuer l'intégration temporelle des deux vitesses ainsi moyennées avec correction des mouvements d'ensemble pour en déduire des valeurs de dilatation D(x,t) des parois, et des courbes de dilatation artérielle le long de chacune des L lignes de la trame, ces courbes étant calées sur la période du cycle cardiaque,

d) des moyens déphaseurs, pour effectuer le recalage en phase desdites courbes de dilatation nécessité par les décalages temporels imposés aux L lignes de chaque trame.

En cas de sténose, la paroi artérielle est sévèrement endommagée, ce qui entraîne une forte perturbation de son fonctionnement. Une artère saine se comporte comme une ligne de propagation grâce à son élasticité propre. Lorsque des plaques ou des sténoses apparaissent en divers endroits, cette élasticité est altérée et le phénomène de propagation est disloqué en conséquence. Un but recherché selon l'invention est d'évaluer la répercussion de la maladie de la paroi artérielle sur son fonctionnement en ligne de propagation, au moyen d'une analyse de la dilatation artérielle pendant le cycle cardiaque. Comme la matière vivante qui constitue l'artère est élastique, il existe une relation linéaire au premier ordre entre les variations du diamètre de l'artère et celles de la pression. Cette considération justifie la mesure par voie externe du diamètre artériel plutôt que celui, invasif, de la pression artérielle.

Le débit sanguin, et la pression (ou plutôt le diamètre artériel) sont des paramètres conjugués qui se propagent avec la même vitesse de propagation. Les deux équations qui décrivent les relations entre débit et pression dans un tube élastique (sans frottement) sont les suivantes :

$$S \frac{dV}{dx} + \frac{dS}{dt} = 0 \qquad (1)$$

$$\rho \frac{dV}{dt} + \frac{dP}{dx} = 0 \qquad (2)$$

où :

S est la section utile de l'artère

V est la vitesse du sang

P est la pression sanguine

x est la distance selon l'axe du vaisseau

t est le temps

$\rho$ est la densité volumique du sang ($10^3$ kg/$m^3$),

la relation (1) exprimant la conservation de la masse, c'est-à-dire du volume sanguin, et la relation (2) étant la version hydraulique de l'équation fondamentale de la dynamique.

La caractéristique d'élasticité de l'artère peut s'exprimer par la compliance C définie par :

$$C = \frac{dS}{dP} \qquad (3)$$

ce qui correspond à l'aptitude que possède l'artère à changer de taille lorsque la pression varie. On montre que la vitesse de propagation c est directement reliée à la compliance comme suit :

$$C = \frac{S}{\rho c^2} \qquad (4)$$

comme décrit dans l'ouvrage : "Mc Donald's Blood Flow in Arteries - troisième édition page 85 : wave propagation in elastic tube, par Wilmer W. Nichols et Michael F. O'Rourke - Edward Arnold éditeur".

La relation (4) montre qu'une rigidité accrue accélère la propagation en même temps qu'elle réduit l'amplitude de la dilatation artérielle.

Par combinaison des relations (3), (4) et (1) on obtient une nouvelle relation :

$$(\rho c^2) \frac{dV}{dx} + \frac{dP}{dt} = 0 \qquad (5)$$

En éliminant le paramètre P entre les équations (5) et (2) et en considérant que, localement, l'élasticité et, en conséquence, la vitesse de propagation c est rendue variable à cause d'une plaque ou d'une sténose, on obtient finalement :

$$\frac{d_2V}{dt^2} - 2c\left(\frac{dc}{dx}\right)\left(\frac{dV}{dx}\right) - c^2\frac{d_2V}{dx^2} = 0 \qquad (6)$$

qui est l'équation différentielle bien connue d'un pavillon exponentiel (dans le domaine acoustique). Naturellement, la perturbation est ici considérée comme une constante dans la sténose, en première approximation, soit : $dc/dx = C^{te}$. La résolution de l'équation (6) fait apparaître une pulsation de coupure $\omega_c$ qui s'exprime par :

$$\omega_c = \left|\frac{dc}{dx}\right| \qquad (7)$$

Ainsi, deux solutions types apparaissent :

- des ondes stationnaires amorties pour $\omega < \omega_c$ :

$$V = e(-mx).[Ae(a(\omega)x) + Be(-a(\omega)x)].e(j(\omega)t)$$

avec :

$$m = \left|\frac{\omega_c}{c}\right|, a(\omega) = m\sqrt{(1-(\frac{\omega}{\omega_c})^2)}$$

- des ondes progressives amorties pour $\omega > \omega_c$ :

$$V = e(-mx).[A.e(j\omega(t-x/c(\omega))) + B.e(j\omega)(t+x/c(\omega)))]$$

avec :

$$m = |\omega_c/c|,\ c(\omega) = c[1-(\omega_c/\omega)^2] - 1/2.$$

De cette analyse, on peut faire l'extrapolation que dès que la perturbation d'élasticité est assez élevée pour provoquer une pulsation de coupure $\omega_c$ du même ordre que la pulsation cardiaque, cette dernière ne se propage plus dès lors à travers la zone perturbée. Qui plus est, lorsque la pulsation est plus élevée que la limite $\omega_c$, la vitesse de propagation dépend de la valeur de la pulsation qui correspond à une propagation dispersive. Pour ce qui est des ondes stationnaires, leur amortissement dépend aussi de leur pulsation : l'amortissement croît avec la fréquence. On peut tracer les courbes de vitesse de propagation et d'amortissement correspondantes en fonction de la fréquence.

Des applications numériques simples peuvent aider pour l'évaluation des effets produits par l'angustie. On suppose par exemple, en premier lieu, une variation de vitesse de propagation axiale de :

$$m = \omega_c/c = (dc/dx)/c = 1\ \%\ \text{par mm},$$

soit une légère perturbation. Pour une vitesse de propagation typique (artère carotide) : c = 6 m/s on trouve :

$$\omega_c = 2\pi fc = 60\ \text{rd/s, soit : } fc = 10\ \text{Hz}.$$

Ceci signifie que dans ce cas, toutes les fréquences inférieures à 10 Hz sont piégées dans la sténose. L'onde associée à la fréquence : fc = 10 Hz est atténuée de : $m = 10^{-2}$/mm. Une plaque de 1 cm de longueur provoque une atténuation : $e^{-mx} = 0,90$.

Pour une sévère perturbation telle que : m = 50 %/mm, et une vitesse de propagation plus élevée : c = 10 m/s (vieilles artères sujettes à des plaques et rigides), alors : fc = 5 kHz. On peut considérer dans ce

cas qu'il n'y a désormais plus de propagation à travers la plaque et que toutes les fréquences significatives (soit en pratique les 15 premiers harmoniques) sont amorties. Le coefficient d'atténuation prend la forme :

$$m - a(\omega) = m\ \omega/2\ \omega_c$$

ce qui révèle une forte déformation de la forme d'onde à travers la sténose.

Des modes de réalisation de l'invention permettent de mettre en évidence les résultats théoriques indiqués ci-dessus.

Un mode de réalisation préféré du procédé pour la détection et la caractérisation de sténose artérielle, est remarquable en ce que lesdites courbes de dilatation recalées, à l'endroit de la sténose et de ses abords immédiats, sont décomposées en leurs premiers harmoniques du cycle cardiaque les plus significatifs, afin de déterminer une fréquence de coupure de valeur fc par rapport à laquelle la sténose se comporte comme un filtre passe haut, pour l'onde de pression sanguine le long de l'artère.

De façon semblable, un mode de réalisation préféré de l'appareil conçu pour la caractérisation d'une sténose artérielle, est remarquable en ce que ladite station de travail comporte des moyens d'analyse pour analyser lesdites courbes de dilatation artérielle et en déduire une fréquence de coupure fc qui traduit le comportement en filtre passe haut de ladite sténose vis-à-vis de l'onde de pression sanguine.

La description qui suit, en regard des dessins annexés, le tout donné à titre d'exemple, fera bien comprendre comment l'invention peut être réalisée.

La figure 1 représente l'état de l'art pour un échographe ultrasonore du type à traitement du signal dans le domaine temporel, avec une chaîne de traitement pour la mesure de dilatation d'une artère.

La figure 2 est une représentation schématique de la séquence de balayage effectuée par l'échographe pour la mise en oeuvre de l'invention.

La figure 3 est un schéma synoptique de l'appareil selon l'invention.

Les figures 4 et 5 représentent des courbes de dilatation d'un segment d'artère que l'invention permet d'obtenir.

Le schéma de la figure 1 montre un dispositif de mesure ponctuelle de dilatation d'une artère sous l'effet de la pression sanguine. Ce dispositif comprend un transducteur piézoélectrique 10 qui peut être, par exemple, une barrette multiéléments. Un circuit 11 d'émission relié au transducteur 10 assure la formation d'un faisceau ultrasonore d'exploration.

De façon classique, le circuit d'émission 11 comprend un séquenceur composé d'un oscillateur et d'un diviseur de fréquence qui commande à la fréquence de récurrence F choisie un générateur dont les signaux électriques d'excitation sont envoyés vers le transducteur 10 qui les convertit en trains périodiques de signaux impulsionnels ultrasonores. Un séparateur 12 des circuits d'émission 11 et de mesure 20 est inséré entre le transducteur 10 et lesdits circuits 11, 20 et évite l'aveuglement des circuits de mesure par les signaux d'émission.

L'unité 20 de mesure de la vitesse v(t,z) montrée à la figure 1 reçoit, après amplification par un amplificateur 13, un signal $S_n(t,z)$ en provenance du séparateur 12, qui est d'abord stocké dans une mémoire 21, puis traité selon la méthode dite d'intercorrélation décrite dans la demande de brevet européen n° 0 225 667 déjà citée et qui utilise le fait que les signaux ultrasonores rétrodiffusés par une cible en mouvement sont reliés par l'équation suivante :

$$S_{n+1}(t) = S_n(t-\tau)$$

ceci signifie que le signal n+1 est la réplique du signal n précédent à un décalage temporel $\tau$ près. Ce dernier représente le temps supplémentaire nécessaire à l'onde ultrasonore pour parcourir le trajet transducteur-cible-transducteur, d'un tir à l'autre, au même endroit, par exemple la ligne 2 sur la figure 1. Autrement dit :

$$\tau = 2vT/C_{us}$$

où v est la vitesse de la cible et $C_{us}$ la vitesse du son. On voit qu'une mesure de $\tau$ permet de mesurer la vitesse v cherchée.

La fonction d'intercorrélation entre $S_n(t)$ et $S_{n+1}(t)$ définie par :

$$C_{n,n+1}(to,u) = \int_{to}^{to+W} S_{n+1}(t+u)\,S_n(t)\,dt$$

vérifie :

$$C_{n,n+1}(to,u) = c_{n,n}(to,u-\tau)$$

Le temps to est lié à la profondeur d'exploration z par to = $2z/C_{us}$, et W est la largeur de la fenêtre d'intégration.

La fonction $C_{nn}(to,u)$ est une fonction d'autocorrélation, et, de ce fait, est maximale pour u = 0. Ainsi, une mesure du décalage temporel $\tau$, et donc de la vitesse v, peut se faire en cherchant pour quel paramètre u la fonction $C_{n,n+1}(to,u)$ est maximale. A cet effet, la fonction d'intercorrélation est échantillonnée, à un pas d'échantillonnage $\Delta t$, entre $u_{min} = -I\Delta t$ et $u_{max} = I\Delta t$ par pas de 1 de façon à obtenir 2I+1 valeurs de fonctions de corrélation. La valeur maximum de ces 2I+1 valeurs correspondant à u = uo permet de mesurer $\tau$ en utilisant l'égalité $\tau$ = uo. Le calcul des fonctions de corrélation est réalisé par le circuit 22 d'intercorrélation montré à la figure 1.

Afin de s'affranchir des erreurs inhérentes à l'échantillonnage dans la détermination du maximum de la fonction de corrélation, il est possible d'utiliser un circuit 23 de multiplexage-interpolation fournissant à partir des valeurs des fonctions de corrélation une estimée plus précise de la vitesse et de la valeur du pic de corrélation correspondant. La demande de brevet européen n° 0 225 667 donne un exemple de ce type de traitement du signal échographique dans lequel la corrélation entre signaux est une corrélation dite "1 bit" en ce sens que les signaux $S_{n+1}$ et $S_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

Contrairement aux vélocimètres Doppler traditionnels, la vitesse d'écoulement v(t,z) ainsi déterminée présente l'avantage d'être insensible à la dispersion de la fréquence de l'onde ultrasonore utilisée, ce qui permet d'envisager une exploitation beaucoup plus complète des résultats.

Les valeurs trouvées pour la vitesse v(t,z) sont stockées dans une mémoire 24 de stockage en vue de traitements ultérieurs.

Pour le calcul précis de la dilatation D(t) de l'artère 1 selon la ligne de tir 2, on effectue d'abord une mesure de l'épaisseur des parois $d_1(t)$ et $d_2(t)$ diamétralement opposées. Cette mesure met en oeuvre, en sortie de l'amplificateur 13 un circuit 31 de calcul de l'énergie locale :

$$A(t,z) = \sum_W |S(t,z)|$$

et un circuit 32 de calcul des épaisseurs de parois $d_1(t) = z_4(t) - z_3(t)$ et $d_2(t) = z_6(t) - z_5(t)$ constitué par un détecteur de seuil de valeur $A_0$ ajustable. Pour éviter toute anbiguïté sur l'emplacement des parois, on peut prévoir d'afficher sur un écran, non représenté, une image du vaisseau 1 étudié, selon la représentation dite en "mode M", de l'évolution au cours du temps du profil de vitesse des parois du vaisseau et prévoir que l'utilisateur, muni d'un stylo optique (souris) localise sur l'écran un point à l'intérieur de chaque paroi, point à partir duquel peuvent alors s'effectuer les sommations (intégrations) qui suivent, en direction des bornes $z_3$ et $z_4$, respectivement $z_5$ et $z_6$. Le codage de la vitesse en mode M est celui utilisé dans les systèmes CFM (Color Flow Mapping) : par exemple le rouge code un sens de déplacement, le bleu le sens inverse et l'intensité de la vitesse est codée par l'intensité de la couleur.

Connaissant, à chaque instant $t_0$, les épaisseurs $d_1(t)$ et $d_2(t)$ des parois, il est possible de calculer les vitesses moyennes $\hat{v}_1(t_0)$ et $\hat{v}_2(t_0)$ des parois grâce à des circuits 36 et 37 constitués chacun par un additionneur et par un diviseur donnant respectivement :

$$\hat{v}_1(t_0) = \Sigma_{d1}\, v_1(t_0,z)/M_1$$

$$\hat{v}_2(t_0) = \Sigma_{d2}\, v_2(t_0,z)/M_2$$

$M_1$ et $M_2$ étant le nombre d'échantillons de mesure sur le segment $[z_3 - z_4]$, respectivement $[z_5 - z_6]$.

Une deuxième intégration (sommation d'échantillons) dans le temps de la vitesse moyenne, permet d'obtenir avec précision la fonction déplacement de chaque paroi, ce qui est réalisé par les additionneurs 38 et 39 :

$$D_1(t) = \sum_{t_0=0}^{t_0=t} \hat{v}_1(t_0)$$

$$D_2(t) = \sum_{t_0=0}^{t_0=t} \hat{v}_2(t_0)$$

On notera que les vitesses des parois, perpendiculaires à celles de l'écoulement sanguin, sont très faibles, de l'ordre de 0,5 cm/s. D'autre part on ne souhaite extraire que les mouvements de sens contraires des parois, c'est-à-dire les mouvements symétriques par rapport au milieu du vaisseau. Il faut donc éliminer les mouvements allant dans le même sens, c'est-à-dire les mouvements antisymétriques dus soit à un déséquilibre des forces de pression de part et d'autre du vaisseau, soit au léger mouvement du transducteur. Le déplacement symétrique moyen s'obtient par la soustraction : $D(t) = D_2(t) - D_1(t)$, dans un soustracteur 41.

L'invention se propose de réaliser au niveau d'une image (d'une trame) ce qui a été décrit ci-dessus avec référence à la figure 1 pour une ligne de tir perpendiculaire aux parois d'une artère, notamment pour un segment d'artère tel que 1 qui comporte une plaque 3 créant localement un rétrécissement de la section utile pour le passage du sang (dans la direction $\vec{x}$ ).

Il s'agit d'utiliser le mouvement de la paroi artérielle pour visualiser et évaluer des perturbations causées à la propagation des ondes dont l'artère est le siège. Il devient ainsi possible de mettre en évidence la dilatation artérielle de façon non invasive, dilatation qui est reliée à la pression grâce à la Compliance. Cependant, ceci implique un procédé de balayage qui doit être compatible avec les contraintes de temps impliquées par le phénomène de propagation des ondes ultrasonores. La séquence de balayage est alors la suivante : la barrette de transducteurs 14 (figure 1) étant disposée parallèlement à l'artère 1, un plan de tir 4 passant par l'axe 5 de l'artère est balayé et enregistré avec une vitesse de trame élevée de l'ordre d'une centaine de Hz, ce qui est possible avec l'échographe représenté à la partie haute de la figure 1, moyennant d'utiliser une trame comportant un nombre L réduit de lignes, de l'ordre d'une centaine (par exemple 128 lignes de tir), et de n'effectuer qu'un seul tir par ligne d'image, ce qui est réalisé par des premiers moyens 15 dans le circuit d'émission 11 (figure 1), étant à la portée de l'homme du métier. Pour une fréquence ligne habituelle de 15 kHz, il en résulte une fréquence trame de l'ordre de 10 ms, soit un nombre d'images MI de l'ordre de 100 pour la durée d'un cycle cardiaque $T_c$.

Une procédure d'intercorrélation semblable à celle décrite ci-dessus en référence à l'unité de mesure 20 est effectuée entre les lignes récurrentes des images successives, comme représenté à la figure 2. Sur cette figure, la ligne 25, située à la cote $x_0$ sur l'axe $\vec{x}$ et choisie comme exemple parmi les L lignes de la trame, équivaut à la ligne 2 de la figure 1, avec une durée entre tirs adjacents plus grande, égale à la durée de trame T. Dans ces conditions, les intercorrélations sont ensuite effectuées entre les lignes 25-1 et 25-2, puis 25-2 et 25-3, et ainsi de suite jusqu'à épuisement des MI images de la séquence d'images, ceci étant effectué pour chacune des L lignes d'image.

En pratique, pour la mise en oeuvre de l'invention, on utilise un échographe pour l'acquisition des données et une station de travail pour le traitement de ces données, comme représenté à la figure 3.

Obtenu par le balayage décrit aux paragraphes précédents, le signal courant $S_n$ en provenance de l'amplificateur 13 sous la forme d'échantillons numériques à une fréquence fe de l'ordre de 20 MHz, est fourni à des moyens d'intercorrélation 51 (de préférence un corrélateur 1 bit), directement, et par l'intermédiaire d'une ligne à retard 52 en parallèle, qui retarde le signal $S_n$ d'une durée de trame T. Le signal intercorrélé en sortie du corrélateur 51 est à son tour fourni à un interpolateur 53, soit directement, soit, comme représenté à la figure 3, par l'intermédiaire de moyens de lissage de signal 54, décrits plus loin. Le circuit 53 fournit à une mémoire 55 de séquence d'images en $\vec{x}$ et $\vec{z}$ , sur au moins la durée $T_c$ d'un cycle cardiaque, des valeurs de vitesses radiales v(x,z,t) du segment d'artère 1 analysé.

Le signal courant $S_n$ est aussi fourni, dans une deuxième chaîne de traitement à un circuit 56 de calcul d'amplitude A, qui réalise par exemple la fonction :

$$A = \sum_{W} |S_n|$$

W étant la largeur temporelle de la fenêtre d'intercorrélation, et le signal A(x,z,t) est aussi fourni à une deuxième mémoire 57 soit directement, soit par l'intermédiaire de moyens de lissage de signal 58 analogues aux moyens 54. La mémoire 57 est, pour les amplitudes des signaux d'écho des points d'images, le pendant de ce qu'est la mémoire 55 pour les vitesses de déplacement mesurées de ces mêmes points. En pratique, la première et seconde mémoire peuvent être constituées par deux zones différentes d'une mémoire unique plus grande. Les moyens de lissage de signal 54 ou 58 ont pour but de réaliser une moyenne glissante des valeurs mesurées pour les points homologues de chaque ligne (tels que 26-1, 26-2, 26-3, ... figure 2) sur un nombre prédéterminé réduit d'images, par exemple 4 images consécutives. La fonction à réaliser, à la portée de l'homme de métier quant à sa réalisation, consiste à faire la moyenne pour les points 26-1, 26-2, 26-3, 26-4, puis pour les points 26-2, 26-3, 26-4, 26-5, et ainsi de suite, jusqu'à la fin de la séquence des MI images.

Les valeurs emmagasinées dans les mémoires 55 et 57 sont fournies à une station de travail 60, par exemple du type SUN SPARC 10 fabriqué par la société américaine SUN MICROSYSTEM, dans laquelle un microprocesseur effectue les opérations suivantes, pour chacune des L lignes d'image :

- en 62, le seuillage des amplitudes A(x,z,t) selon $\vec{z}$ afin d'identifier les 2 parois artérielles, comme décrit ci-dessus en référence au circuit 32 de la figure 1,
- en 66 et 67, le moyennage spatial selon $\vec{z}$ de l'information de vitesse v(x,z,t) dans chaque paroi,
- en 68 et 69, l'intégration temporelle des vitesses moyennes dans chaque paroi, pour fournir pour chaque ligne d'image et à chaque instant du cycle cardiaque, une valeur précise de la dilatation artérielle D(x,t), en sortie d'un mélangeur 71.

A noter que la séquence d'images de dilatation d'artère que l'on souhaite visualiser consisterait en une succession d'images instantanées prises pendant le déroulement du cycle cardiaque. Or, le balayage trame pratiqué dans la direction $\vec{x}$ , selon l'invention, est de l'ordre de quelques mètres par seconde, soit du même ordre de grandeur que la vitesse de propagation de l'onde de pression le long de l'artère. Il est donc impératif, pour ne pas fausser gravement la visualisation du mouvement du vaisseau, de recaler en phase les valeurs D(x,t) obtenues, pour chaque valeur de x, ce qui est facile à réaliser, étant donné que les décalages temporels entre lignes sont bien connus, en raison d'une fréquence ligne constante (par exemple 15 kHz). A cet effet, un circuit 72 est prévu pour fournir des signaux recalés D'(x,t) ; on peut par exemple opérer dans le plan fréquentiel par décomposition en harmoniques du signal D(x,t), recalage en phase des harmoniques et reconstruction du signal par recomposition à partir des harmoniques recalés les plus significatifs, par exemple les 15 premiers.

Outre le rattrapage de phase précité, il convient d'ajuster les valeurs de dilatation D(x,t) à visualiser, sur l'écran d'un moniteur 73, entre le début et la fin du cycle cardiaque $T_c$. En effet, la dilatation est supposée nulle au début et à la fin du cycle cardiaque. Pour cela, le déroulement du cycle doit être connu, soit par le biais d'un électrocardiogramme effectué en même temps que les mesures sur l'artère, soit à partir des dilatations de l'artère elle-même, D'(x,t) (de façon non représentée, à la portée de l'homme du métier).

Les valeurs des courbes de dilatation que l'on obtient sont positives lorsqu'il y a une dilatation et négatives lorsqu'il y a une contraction. Ainsi sont représentées les courbes de la figure 4, pour un segment d'artère carotide de 2,5 cm de longueur, comportant une petite plaque, telle que 3 sur les figures 1 et 2. La représentation adoptée est celle utilisée habituellement en dessin industriel, avec vue de face selon le plan $\vec{x}$ , $\vec{t}$ , vue de droite selon le plan $\vec{z}$ , $\vec{t}$ , et vue de dessous selon le plan $\vec{x}$ , $\vec{z}$ . Sur la vue de dessous, les points centraux matérialisent les barycentres de certains profils de dilatation z(t).

Dans l'exemple de la figure 4, la plaque perturbatrice est jugée non pathologique par les médecins. Dans le plan $\vec{z}$ , $\vec{t}$ sont représentées les courbes de dilatations en fonction du temps, toutes les courbes qui correspondent à la direction d'exploration étant superposées. Dans le plan $\vec{x}$ , $\vec{z}$ figurent les excursions des mêmes courbes de dilatation (entre maximum et minimum pour chaque courbe) en fonction de la position de balayage transversal (en $\vec{x}$ ). Les résultats spectaculaires que traduisent ces courbes révèlent un étonnant comportement qui n'est pas décrit dans les dossiers médicaux, probablement parce qu'il n'était pas encore possible de le mettre en évidence : en fait, l'artère commence par se contracter, au lieu de se dilater, juste en aval de la plaque, repérée par la référence PL. La contraction est de l'ordre de 150 µm alors que la dilatation en amont est d'environ 300 µm. Une analyse plus détaillée compatible avec l'analyse mathématique exposée ci-dessus est une décomposition en harmoniques. Ceci est représenté à la figure 5

où l'on montre, selon les mêmes conventions que sur la vue dans le plan $\vec{x}$ , $\vec{t}$ de la figure 4 (déplacement total), l'harmonique 1 ou fondamental, qui n'est pas transmis à cause de la plaque (soit : $fc > 1/T_c$), et l'harmonique 2 qui est transmis, ainsi que les suivants, non représentés (soit : $fc < 2/T_c$). La ligne continue, sur les figures dans le plan $\vec{x}$ , $\vec{t}$ (figures 4 et 5), marque les points de dilatation nulle, ce qui constitue une frontière entre les zones de dilatation (parties hachurées et indiquées +) et les zones de contraction (parties claires et indiquées -). Si l'on observe les différentes perturbations dans la propagation de ces fréquences, il est clair que l'harmonique 1 qui correspond à la fréquence cardiaque ne se propage pas à travers la plaque : une inversion de signe apparaît le long de l'axe $\vec{x}$ , caractéristique d'ondes stationnaires. Les fréquences élevées réussissent à traverser la plaque, même si les amplitudes des déplacements sont un peu atténuées. Dans ce cas, la fréquence de coupure est donc comprise entre les premier et deuxième harmoniques, plus précisément entre 1 et 2 Hz. La perturbation locale peut alors être évaluée seulement à :

$$\frac{dc}{dx} = 10\ s^{-1} = 10^{-2}\ (\frac{m/s}{mm})$$

ce qui semble être négligeable et en accord avec le diagnostic de l'Angiologue.

A partir des données de vitesse emmagasinées dans la mémoire 55, il est aussi intéressant et d'un grand enseignement de calculer les déplacements de tous les points sondés puis d'en déduire des images de compression des milieux biologiques traversés.

Pour ce mode de réalisation de l'invention, l'appareil de la figure 3 comporte une chaîne de traitement supplémentaire dans la station de travail 60, reliée à la sortie de la mémoire 55 :

- en premier lieu, les vitesses v(x,z,t) sont intégrées par rapport au temps, en 81, ce qui fournit des déplacements d(x,z,t) des points des tissus traversés,
- puis des calculs de gradient en z de ces déplacements résultent en des données de compression de ces tissus, soit $d'_z(x,z,t)$, ce qui permet d'obtenir des images de compression sur l'écran du moniteur 73.

Sur le moniteur de la station de travail, il est possible d'obtenir une représentation cinématique du phénomène de déformation, avec ralentissement du temps, de préférence, dans un rapport de l'ordre de 5 par rapport au temps d'acquisition.

Ceci permet, dans une zone d'angustie de l'artère, de distinguer une plaque fibreuse dure d'un athérome mou notamment.

**Revendications**

**1.** Procédé de détection et/ou de caractérisation d'un segment d'artère par échographie ultrasonore à partir d'une barrette de transducteurs ultrasonores, engendrant un plan de coupe ou trame constitué par L lignes de tir parallèles, successives dans une direction $\vec{z}$ perpendiculaire à l'axe $\vec{x}$ de l'artère, associée à un circuit d'émission et à un circuit de réception, ladite échographie étant du type à traitement de signal dans le domaine temporel avec intercorrélation des signaux échographiques relatifs auxdites lignes et interpolation des signaux intercorrélés pour la détermination des vitesses radiales v des points dudit plan de coupe limités à la traversée des parois de l'artère et à ses environs immédiats, caractérisé en ce que, ladite trame étant balayée selon une période T de quelques ms, à raison d'un tir par ligne, lesdites intercorrélation et interpolation sont effectuées entre lignes de tir colinéaires décalées de la période T, afin d'obtenir au moins pour la durée d'un cycle cardiaque, dans une première mémoire une première séquence d'images en $\vec{x}$ et $\vec{z}$ de valeurs de vitesse v(x,z,t), dans une deuxième mémoire une deuxième séquence d'images en $\vec{x}$ et $\vec{z}$ de valeurs d'amplitudes A(x,z,t), et que lesdites valeurs de vitesse et d'amplitude sont ensuite exploitées sur station de travail pour en déduire, par intégration temporelle des valeurs de vitesses, la courbe de dilatation artérielle le long de chacune des L lignes de trame pendant au moins la durée Tc d'un cycle cardiaque.

**2.** Procédé de détection et de caractérisation d'un segment d'artère selon la revendication 1, caractérisé en ce que les opérations suivantes sont effectuées par la station de travail :

a) le seuillage des amplitudes pour identifier les 2 parois artérielles diamétralement opposées,

b) le moyennage spatial selon $\vec{z}$ des vitesses dans chaque paroi,

c) l'intégration temporelle des deux vitesses ainsi moyennées avec correction des mouvements d'ensemble pour en déduire des valeurs de dilatation D(x,t) des parois, et des courbes de dilatation artérielle le long de chacune des L lignes de la trame, ces courbes étant calées sur la période Tc du

cycle cardiaque,
d) le recalage en phase desdites courbes de dilatation nécessité par les décalages temporels imposés aux L lignes de chaque trame.

**3.** Procédé selon la revendication 1 ou 2, caractérisé en ce qu'il comporte en outre une étape supplémentaire de calcul de moyenne glissante du signal entre les phases d'intercorrélation et d'interpolation 1 bit ainsi qu'une étape supplémentaire de calcul de moyenne glissante du signal représentatif des amplitudes A(x,z,t) avant inscription dans ladite deuxième mémoire.

**4.** Procédé selon l'une des revendications 1 à 3, pour la détection et la caractérisation de sténose artérielle, caractérisé en ce que lesdites courbes de dilatation recalées, à l'endroit de la sténose et de ses abords immédiats, sont décomposées en leurs premiers harmoniques du cycle cardiaque les plus significatifs, afin de déterminer une fréquence de coupure de valeur fc par rapport à laquelle la sténose se comporte comme un filtre passe haut, pour l'onde de pression sanguine le long de l'artère.

**5.** Procédé de détection et de caractérisation de sténose selon l'une des revendications 1 à 4, caractérisé en ce qu'il comporte l'étape supplémentaire de calcul du gradient dans la direction $\vec{z}$ des valeurs d'élongation d(x,z,t), obtenues par intégration temporelle des vitesses v(x,z,t) contenues dans ladite première mémoire et permettant de produire, dans une dernière étape, des images de compression pour les parois du segment d'artère analysé et des tissus adjacents.

**6.** Appareil pour la détection et la caractérisation d'un segment d'artère comportant un échographe ultrasonore et une station de travail, l'échographe consistant en une sonde munie d'une barrette de transducteurs ultrasonores, conçue pour émettre une trame de L lignes successives selon une période T dans une direction $\vec{z}$ perpendiculaire à l'axe $\vec{x}$ de l'artère, un circuit d'émission et un circuit de réception et étant du type à traitement de signal dans le domaine temporel et comportant à cet effet des moyens d'intercorrélation et des moyens d'interpolation des signaux échographiques, caractérisé en ce que ledit circuit d'émission comporte des premiers moyens pour effectuer un seul tir par ligne de trame, que ledit circuit de réception comporte des deuxièmes moyens pour effectuer lesdites intercorrélations et interpolations entre lignes de tirs colinéaires décalées de la période T, qu'il comporte en outre une première mémoire de séquence d'images en $\vec{x}$ et $\vec{z}$ de valeurs de vitesses v(x,z,t) et une deuxième mémoire de séquences d'images en $\vec{x}$ et $\vec{z}$ de valeurs d'amplitudes A(x,z,t), pendant la durée d'au moins un cycle cardiaque, et que ladite station de travail comporte des moyens pour exploiter le contenu desdites première et deuxième mémoires et en déduire des valeurs d'élongation ainsi que des courbes de dilatation pour le segment d'artère analysé.

**7.** Appareil selon la revendication 6, caractérisé en ce que ledit circuit de réception comporte en outre des moyens de lissage de signal disposés d'une part entre lesdits moyens d'intercorrélation, qui sont des moyens d'intercorrélation 1 bit, et d'interpolation et d'autre part des moyens de lissage du signal d'amplitude, disposés juste en amont de ladite deuxième mémoire.

**8.** Appareil selon la revendication 6 ou 7, caractérisé en ce que ladite station de travail comporte :
a) des moyens de seuillage pour seuiller lesdites amplitudes A(x,z,t) selon $\vec{z}$ afin d'identifier les 2 parois artérielles,
b) des moyens de moyennage pour effectuer un moyennage spatial selon $\vec{z}$ de l'information de vitesse v(x,z,t) dans chaque paroi,
c) des moyens d'intégration pour effectuer l'intégration temporelle des deux vitesses ainsi moyennées avec correction des mouvements d'ensemble pour en déduire des valeurs de dilatation D(x,t) des parois, et des courbes de dilatation artérielle le long de chacune des L lignes de la trame, ces courbes étant calées sur la période du cycle cardiaque,
d) des moyens déphaseurs, pour effectuer le recalage en phase desdites courbes de dilatation nécessité par les décalages temporels imposés aux L lignes de chaque trame.

**9.** Appareil selon la revendication 8, conçu pour la caractérisation d'une sténose artérielle, caractérisé en ce que ladite station de travail comporte des moyens d'analyse pour analyser lesdites courbes de dilatation artérielle et en déduire une fréquence de coupure fc qui traduit le comportement en filtre passe haut de ladite sténose vis-à-vis de l'onde de pression sanguine.

**10.** Appareil selon l'une des revendications 6 à 9, caractérisé en ce que ladite station de travail comporte des moyens d'intégration pour effectuer l'intégration temporelle des vitesses v(x,z,t) et en déduire des valeurs d'élongation d(x,z,t), et des moyens de calcul pour calculer le gradient dans la direction $\vec{z}$ desdites valeurs d'élongation d(x,z,t) et en déduire des images de compression pour les parois du segment d'artère analysé et des tissus adjacents.

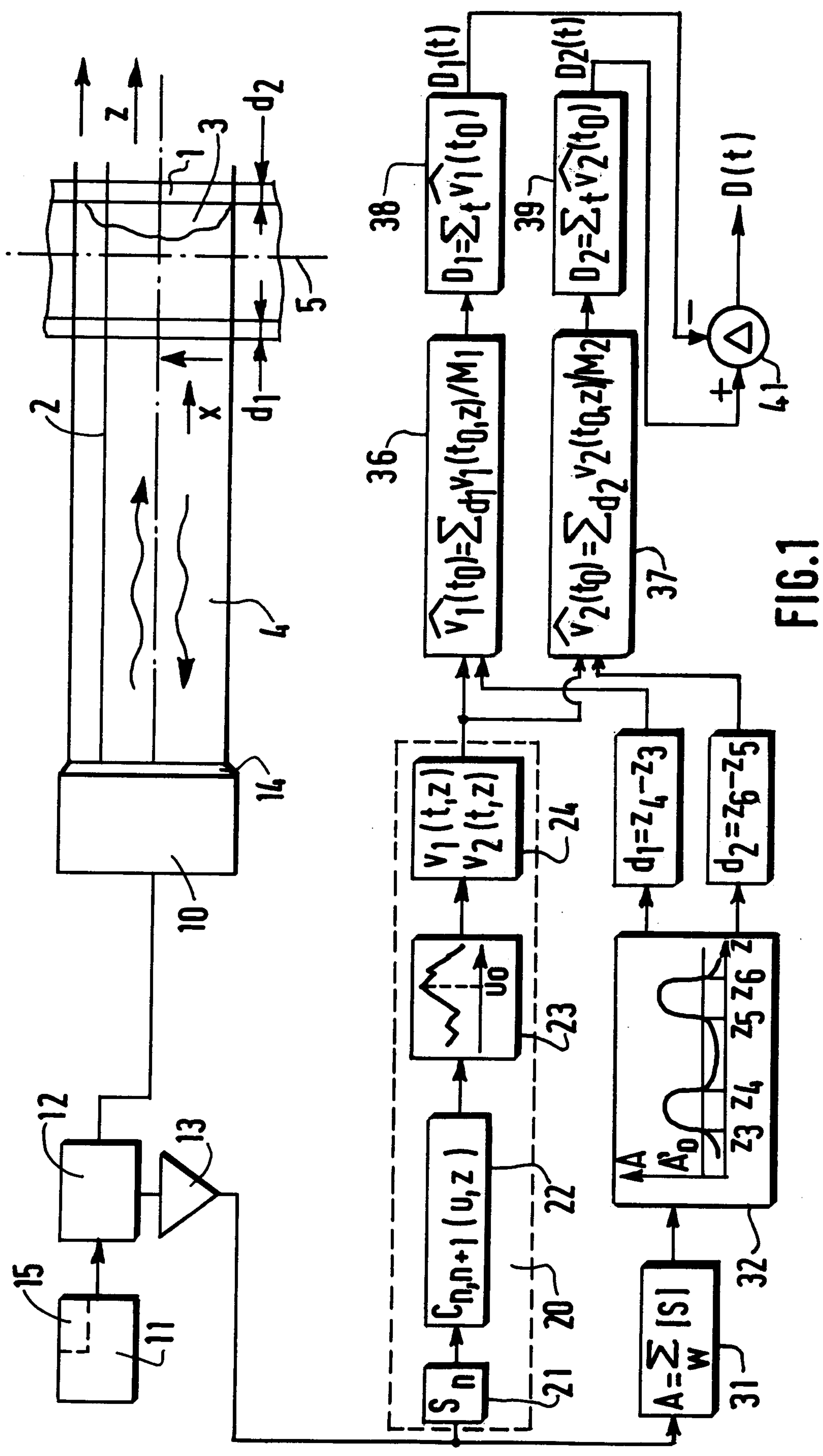
1
2
3
4
5
10
11
12
13
14
15
20
21
22
23
24
31
32
36
37
38
39
41
z
x
d1
d2
D1(t)
D2(t)
D(t)
Sn
Cn,n+1(u,z)
v1(t,z)
v2(t,z)
u0
A=Σ|S|
W
d1=z4−z3
d2=z6−z5
z3 z4 z5 z6
z
A
Â0
+
−

FIG.1

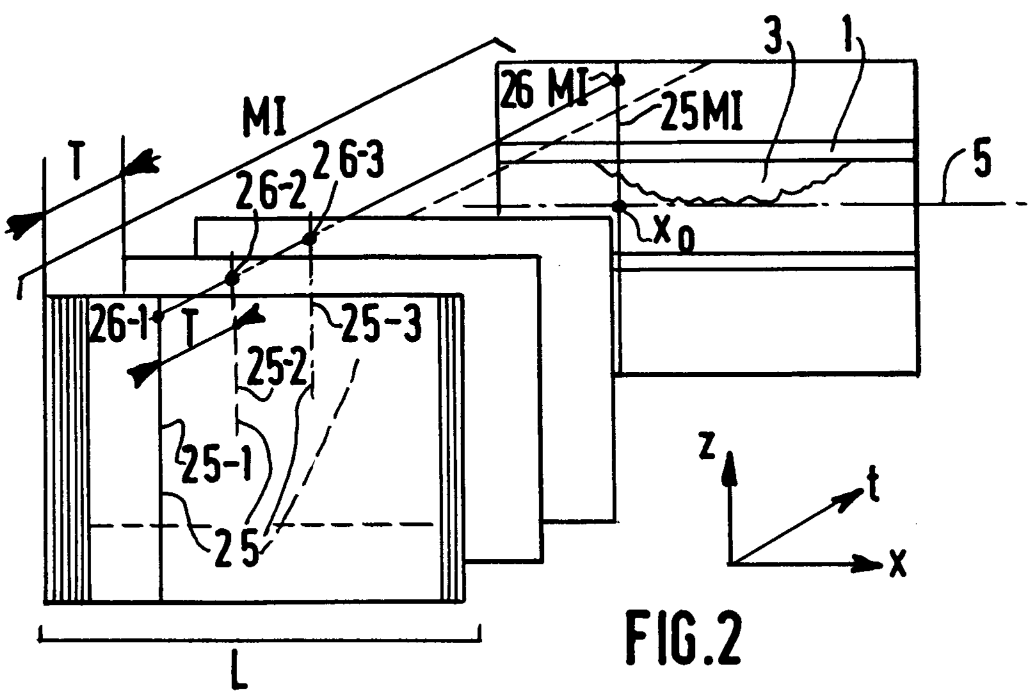
MI
T
26-2
26-3
26 MI
25MI
3
1
5
x0
26-1
T
25-3
25-2
25-1
25
z
t
x
L

FIG.2

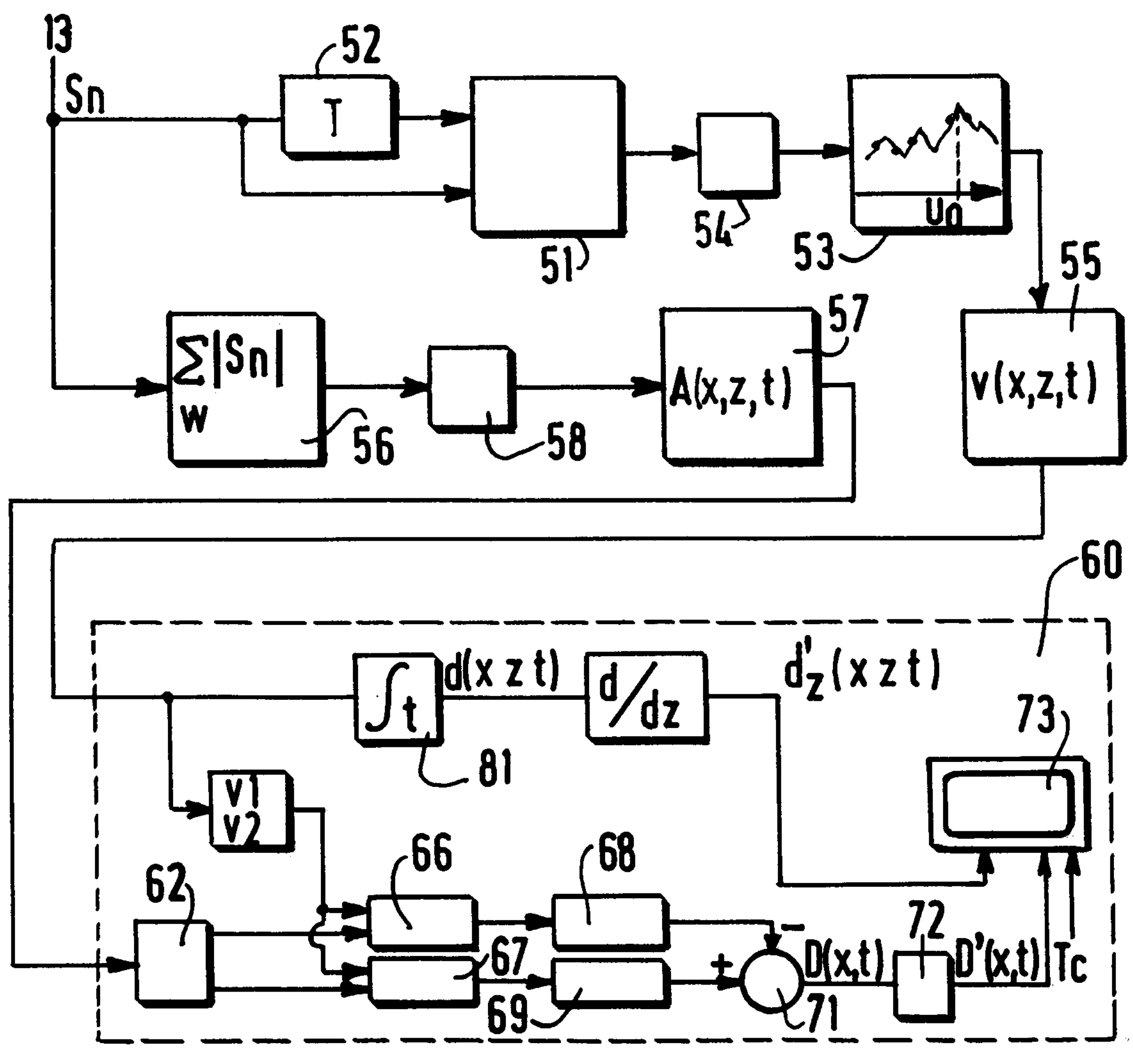
13
Sn
52
T
51
54
53
Un
55
∑|Sn|
W
56
58
A(x,z,t)
57
v(x,z,t)
60
∫t
d(x z t)
d/dz
d'z(x z t)
81
73
v1
v2
62
66
68
67
69
D(x,t)
71
72
D'(x,t)
Tc

FIG.3

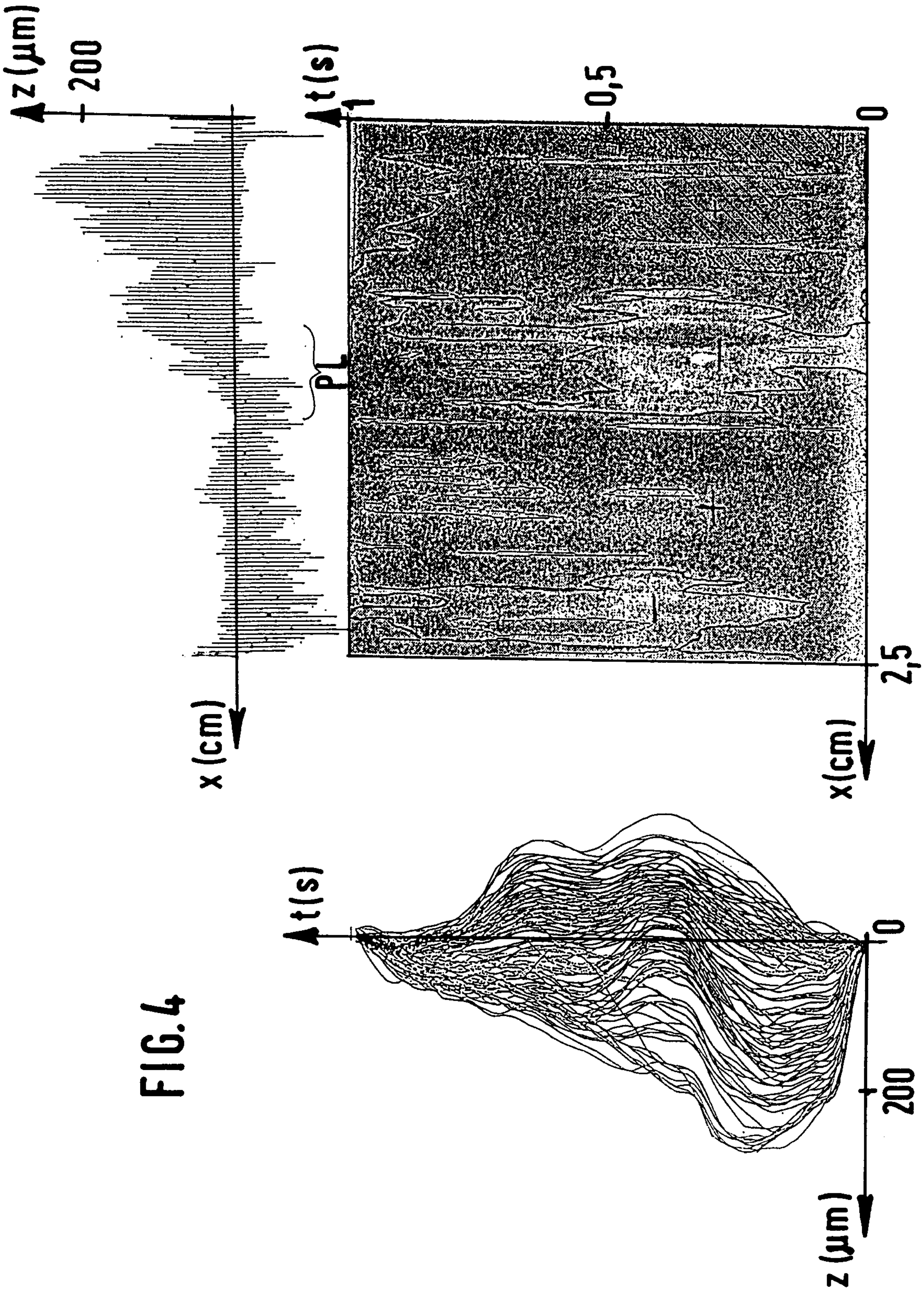


FIG. 4

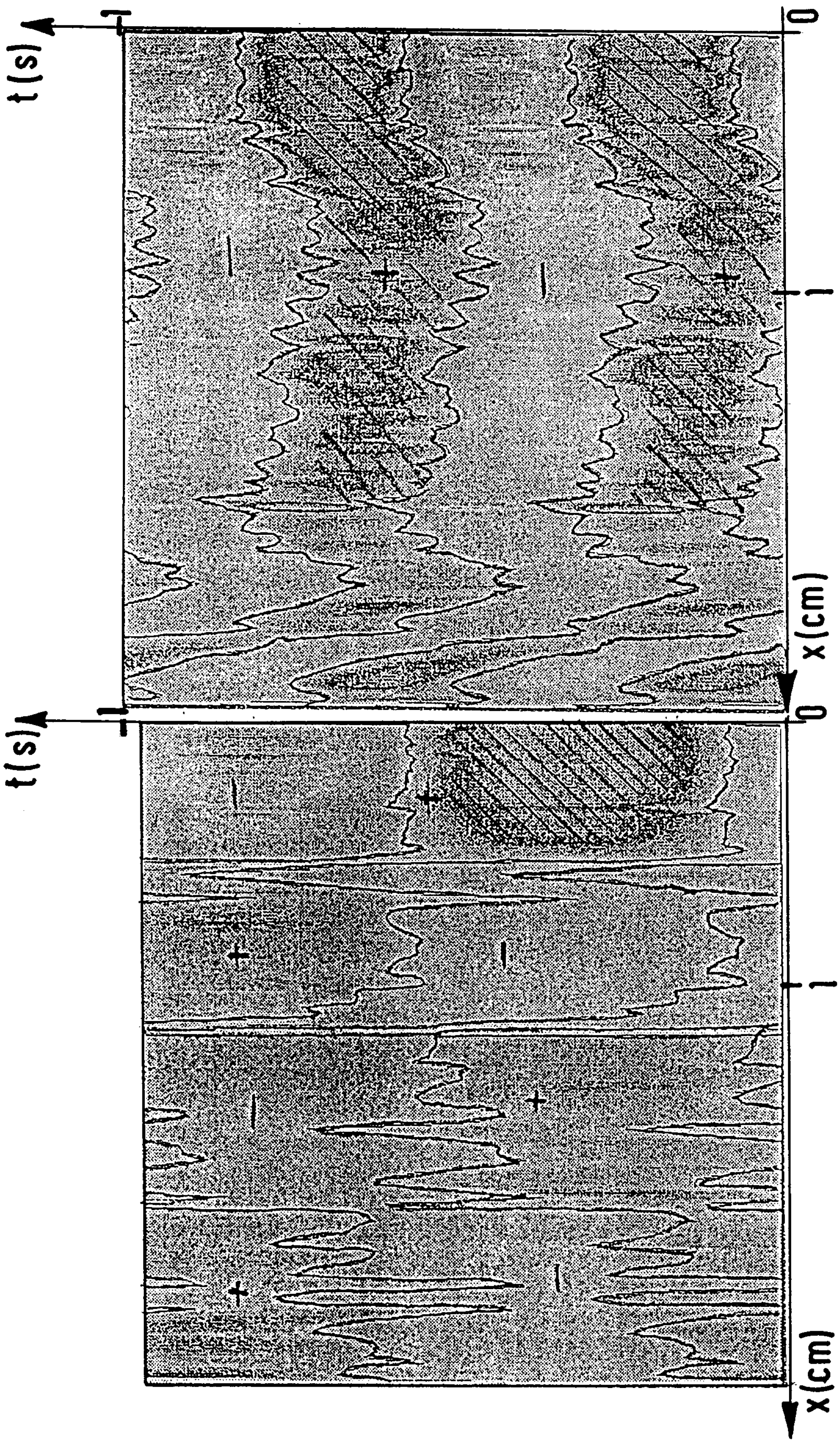


FIG.5

Office européen des brevets

# RAPPORT DE RECHERCHE EUROPEENNE

Numero de la demande
EP 95 20 0679

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
| --- | --- | --- | --- |
| X | EP-A-0 458 384 (PHILIPS) 27 Novembre 1991<br>* le document en entier *<br>--- | 1,2,6,8 | G01S15/89<br>A61B8/06 |
| A | FR-A-2 591 884 (WASHINGTON RESEARCH FOUNDATION) 26 Juin 1987<br>* abrégé *<br>--- | 4,9 | |
| A | WO-A-91 11146 (COMMONWEALTH SCIENTIFIC AND INDUSTRIAL RESEARCH ORGANISATION) 8 Août 1991<br>* abrégé *<br>* page 18; figure 4 *<br>--- | 1,6 | |
| P,X | EP-A-0 603 967 (LABORATOIRES D'ELECTRONIQUE PHILIPS) 29 Juin 1994<br>* le document en entier *<br>----- | 1,6 | |

| DOMAINES TECHNIQUES RECHERCHES (Int.Cl.6) |
| --- |
| G01S<br>A61B |

Le présent rapport a été établi pour toutes les revendications

| Lieu de la recherche | Date d'achèvement de la recherche | Examinateur |
| --- | --- | --- |
| LA HAYE | 30 Juin 1995 | Zaccà, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)